# EUROPÄISCHE PATENTSCHRIFT

(11) **EP 0 485 864 B1**
(45) Veröffentlichungstag und Bekanntmachung des Hinweises auf die Patenterteilung: **15.02.1995**
(21) Anmeldenummer: 91118849.8
(22) Anmeldetag: 05.11.1991
(51) Int. Cl.: A61N 5/06, F21V 29/00

(54) **Bestrahlungsvorrichtung**
Irradiation device
Dispositif d'irradiation

(30) Priorität: 16.11.1990 DE 9015721 U
(43) Veröffentlichungstag der Anmeldung: 20.05.1992
(73) Patentinhaber: MAXS AG, CH-6072 Sachseln (CH)
(72) Erfinder: Rzeznik, Jerry, W-6301 Heuchelheim (DE); Braun, Werner, CH-6062 Wilen-Sarnen (CH)
(74) Vertreter: Grünecker, Kinkeldey, Stockmair & Schwanhäusser Anwaltssozietät

(56) Entgegenhaltungen:
- EP-A- 0 311 898
- DE-C- 249 146

## Beschreibung

Die Erfindung bezieht sich auf eine Bestrahlungsvorrichtung mit einer Strahlenquelle und einem im Strahlengang angeordneten Filter, der zwei transparente, im wesentlichen planparallel zueinander angeordnete Scheiben aufweist, die mit ihren umlaufenden Rändern in einem Rahmen aus gut wärmeleitendem Material gehalten sind, wobei die Scheiben und der Rahmen einen geschlossenen Hohlraum begrenzen, in dem ein das Strahlenspektrum selektiv beeinflussendes Medium vorgesehen ist.

Eine solche Bestrahlungsvorrichtung ist in der EP-A-311 898 beschrieben. Dort besteht der Rahmen aus einem gut wärmeleitenden Metall, z.B. Aluminium, während die Scheiben, zumindest zum Teil aus Kunststoff bestehen können. Während bei einer Variante beide Scheiben völlig planparallel zueinander angeordnet sind, ist bei einer anderen Variante die der Strahlungsquelle zugewandte Scheibe konvex nach außen gewölbt. Der Hohlraum zwischen den beiden Scheiben ist mit Wasser gefüllt. Der auf diese Weise erstellte Wasserfilter filtert bestimmte Anteile des Strahlenspektrums heraus, die sogenannten Wasserbanden. Aufgrund der Bestrahlung des Wasserfilters mit einer Halogenlampe werden sowohl die beiden Scheiben als auch das Medium stark erwärmt. Für einen dauernden Betrieb ist es notwendig, die Wärme aus dem Medium heraus nach außen zu transportieren, um eine Zerstörung des Wasserfilters zu vermeiden. Derartige Maßnahmen sind in der EP-A-311 898 ausführlich beschrieben.

Aufgabe der vorliegenden Erfindung ist es, mit einfachen konstruktiven Mitteln die Bestrahlungsvorrichtung auch für den Einsatz noch leistungsstärkerer Strahlenquellen geeignet zu machen.

Diese Aufgabe wird erfindungsgemäß dadurch gelöst, daß zumindest eine der Scheiben topfförmig mit einem abgestuften Rand derart ausgebildet ist, daß der lichte Abstand der im Rahmen gehaltenen Ränder der Scheiben größer ist als der lichte Abstand der mittleren Bereiche der Scheiben.

Während die optischen Eigenschaften des Filters unverändert bleiben, vergrößert sich die Fläche des Rahmens, die mit dem Medium in Berührung kommt. Auf diese verblüffend einfache Weise ist es zum einen möglich, den Wärmefluß aus dem Medium in die Umgebung erheblich zu verbessern, zum anderen wird zwischen dem abgestuften Rand ein zusätzliches Volumen geschaffen, in dem sich etwaige Gasblasen aufhalten können, ohne daß die Wirkung des Filters hierdurch beeinflußt wird. Auf diese Weise können auch leistungsstärkere Strahlenquellen zum Einsatz kommen.

In bevorzugter Weise kann die topfförmige Scheibe auf der der Strahlenquelle abgewandten Seite des Filters vorgesehen sein. Dann ist es möglich, die topfförmige Scheibe auch bei leistungsstärkeren Strahlenquellen aus Kunststoff, vorzugsweise Polycarbonat, herzustellen, da die maximale Temperatur dieser Scheibe durch die Temperatur des Mediums bestimmt wird.

In günstiger Weise kann die Strahlungsquelle auch in einem paraboloidförmigen Reflektor angeordnet sein, der bis nahe an den Filter heranreicht. Ein solcher paraboiloidförmiger Reflektor sorgt für eine gleichmäßige Ausleuchtung des Filters und vermeidet so örtliche Überhitzungen. Wenn der Reflektor, der aufgrund seiner Konstruktion eine sehr große Oberfläche aufweist, auch noch aus Aluminium besteht, kann über die Reflektorwände ein Großteil der von der Strahlenquelle erzeugten Wärme abgeführt werden.

Die der Strahlenquelle zugewandte Scheibe kann in bevorzugter Weise aus einem hitzebeständigen Material, vorzugsweise Mineralglas, bestehen. Wenn die Strahlenquelle nicht ganz so leistungsstark ist, kann auch Polysulfon verwendet werden, ansonsten wird eine unter der Bezeichnung "Robax" gehandelte Glaskeramik bevorzugt. Diese Scheibe kann auch mit einer das sichtbare Spektrum reflektierenden Bedampfungsschicht oder Folie versehen sein, um die thermische Belastung des Filters weiter zu vermindern.

In besonders günstiger Weise kann die Stufe der topfförmigen Scheibe im wesentlichen kegelschnittförmig und zum Rand hin beabstandet ausgebildet sein. Auf diese Weise wird bei einerseits möglichst großer mit dem Medium in Berührung stehender Rahmenfläche auch zugleich ein möglichst großer Bereich beibehalten, der für den Strahlengang als wirksamer Filter zur Verfügung steht.

Günstig scheint es zu sein, wenn der Abstand der Stufe zum Rand etwa zwischen einem Drittel und einem Zehntel des Radius der Scheibe liegt. Auf diese Weise wird einerseits ein guter Wärmefluß erreicht, andererseits steht eine genügend große Fläche für den Strahlendurchgang zur Verfügung.

Bevorzugt wird auch, wenn das Verhältnis des lichten Abstandes der Ränder zum lichten Abstand der mittleren Bereiche der Scheiben etwa zwischen 7:1 bis 3:1 liegt.

Wenn der Rahmen an seiner dem Hohlraum zugewandten Seite mit in das Medium reichenden Kühlrippen versehen ist, läßt sich nicht nur die wirksame Kühlfläche zur Abgabe der Wärme aus dem Medium vergrößern; es werden gleichzeitig auch Luftfallen für etwaige Gasbläschen gebildet.

Wenn man auch die topfförmige Scheibe aus Glas herstellen will, ist es günstig, wenn man zum Druckausgleich eine Druckausgleichseinrichtung in Form einer im Inneren des Hohlraums angeordneten kompressiblen Blase oder eines mit dem Hohlraum verbundenen expandierbaren Schlauchs vorsieht. Auch bei Verwendung von Kunststoffscheiben ist dies günstig, um auf diese Weise eine Linsenbildung durch Auswölbung der Scheibe unter Druck zu vermeiden.

Wenn man die topfförmige Scheibe, die ja in bevorzugter Weise aus Kunststoff bestehen kann, einfärbt, kann die Scheibe auch eine Funktion als Kantenfilter übernehmen, d.h. sie kann je nach Art der Färbung den Spektralbereich begrenzen.

Um bestimmte optische Wirkungen zu erzielen, kann der mittlere Bereich der topfförmigen Scheibe auch als Fresnel-Scheibe ausgebildet sein. Auch hier erweist es sich als günstig, daß die topfförmige Scheibe aus Kunststoff bestehen kann, so daß die Fresnel-Struktur beim Herstellen der Scheibe eingeprägt werden kann.

Im folgenden wird ein Ausführungsbeispiel der Erfindung anhand einer Zeichnung näher erläutert. Es zeigen:
- Fig. 1: in einer geschnittenen Seitenansicht ein Ausführungsbeispiel der Bestrahlungsvorrichtung in schematischer Darstellung,
- Fig. 2: eine Ansicht der Bestrahlungsvorrichtung von rechts,
- Fig. 3: in vergrößerter Ansicht das Detail III aus Fig. 1, und
- Fig. 4: in einer ähnlichen Ansicht wie Fig. 1 eine weitere Ausführungsform der Bestrahlungsvorrichtung mit einem Reflektor in Paraboloidform.

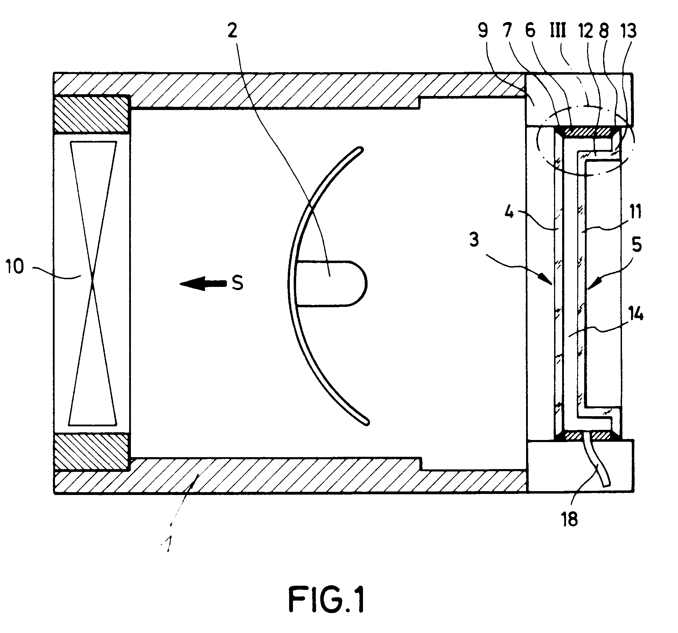

Wie besonders deutlich aus Fig. 1 zu erkennen ist, umfaßt die Bestrahlungsvorrichtung ein rohrförmiges Gehäuse 1, in dessen Innerem eine Strahlungsquelle 2 in Form einer Halogenlampe angeordnet ist.

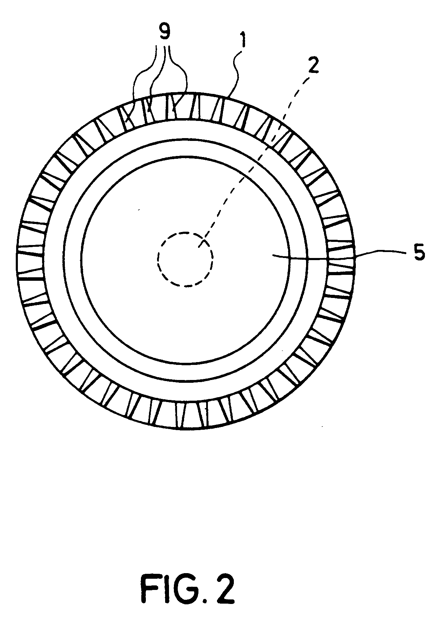

Im Strahlengang hinter der Strahlungsquelle 2 ist ein Filter 3, ein sog. Wasserfilter, angeordnet. Der Filter 3 umfaßt zwei im wesentlichen planparallel zueinander angeordnete Scheiben 4 und 5, die mit ihren Rändern 7 bzw. 8 in einem Rahmen 6 gehalten sind. Wie besonders gut aus Fig. 2 ersichtlich ist, weist der Rahmen 6 an seiner Außenseite radial abstehende Kühlrippen 9 auf, die bis an das Gehäuse 1 der Bestrahlungsvorrichtung heranreichen.

Auf der der Strahlungsquelle abgewandten Seite des Gehäuses 1 ist noch ein Kühlventilator 10 vorgesehen, mit welchem durch die Kühlrippen 9 hindurch Luft durch das Gehäuse 1 gesaugt werden kann, um die thermische Belastung im Inneren der Bestrahlungsvorrichtung auf einem niedrigen Niveau zu halten.

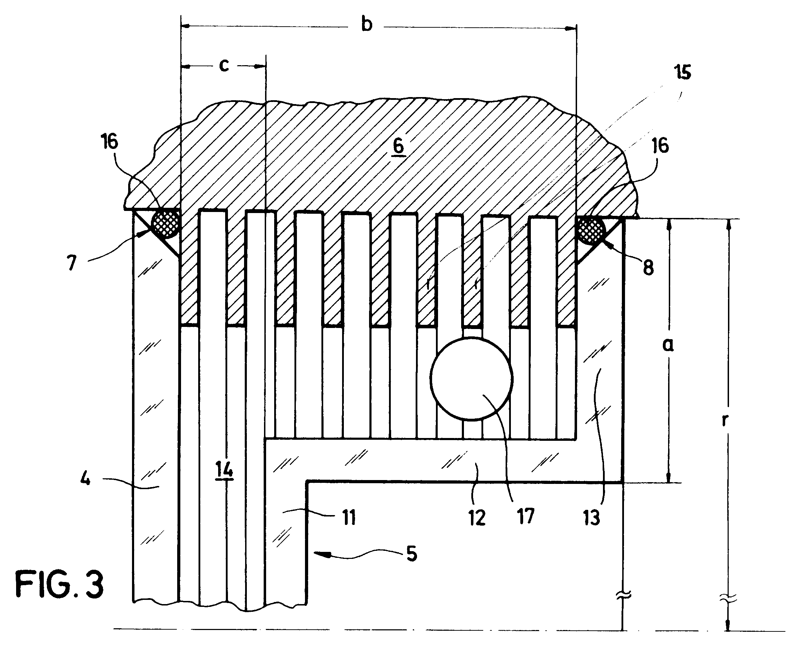

Die der Strahlungsquelle 2 zugewandte Scheibe 4 des Filters besteht aus einem Mineralglas oder Glaskeramik, z.B. Robax, und ist vollkommen plan ausgebildet. Die der Strahlungsquelle 2 abgewandte Scheibe 5 besteht aus Polycarbonat und ist topfförmig ausgestaltet, wobei der mittlere Bereich 11 bei dem hier gezeigten Ausführungsbeispiel planparallel zu der ersten Scheibe 4 verläuft. Der abgestufte Rand besitzt eine Stufe 12, die im wesentlichen zylindermantelförmig ausgebildet ist und die in einen radial nach außen weisenden Abschnitt 13 übergeht, der an dem Rand 8 endet. Die radiale Abmessung a des radial nach außen weisenden Abschnittes 13 entspricht bei dem hier gezeigten Ausführungsbeispiel etwa einem Fünftel des Radius r der Scheibe 5. Der Abstand b der Ränder7, 8 der beiden Scheiben 4 bzw. 5 zueinander beträgt bei dem hier gezeigten Ausführungsbeispiel etwa das Vierfache des Abstandes c der beiden mittleren Bereiche 11 der beiden Scheiben 4 bzw. 5 voneinander. Wie deutlich aus Fig. 3 ersichtlich ist, ist die dem durch die Scheiben 4 und 5 begrenzten Hohlraum 14 zugewandte Seite des Rahmens 6 mit umlaufenden Kühlrippen 15 versehen. Die Abdichtung der Scheiben 4 und 5 gegenüber dem Rahmen 6 erfolgt über O-Ringdichtungen 16.

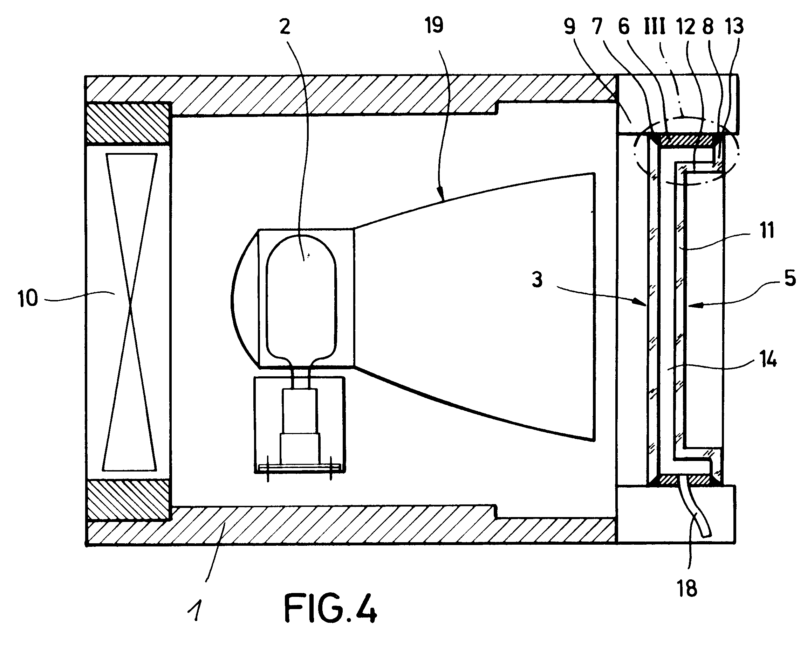

In Fig. 4 ist eine Variante der in Fig. 1 dargestellten Bestrahlungsvorrichtung gezeigt. Dort ist die Strahlungsquelle 2 in einem Reflektor 19 angeordnet, der eine Paraboloidform aufweist. Die Länge des Reflektors 19 entspricht nahezu dem doppelten Durchmesser an der der Scheibe 4 zugewandten Lichtaustrittsöffnung. Im Bereich der Strahlenquelle ist der Reflektor zylindermantelförmig ausgebildet; hinter der Strahlenquelle 2 schließt der Reflektor mit einer Kugelkalotte ab. Die Reflektorwände bestehen aus Aluminium, so daß durch die große Oberfläche des Reflektors 19 ein Großteil der Wärme abgeführt wird. Der Reflektor 19 sorgt für eine gleichmäßige Ausleuchtung, wodurch örtliche Wärmespitzen im Filter vermieden werden. Die Strahlenquelle 2 ist in dem Reflektor 19 so angeordnet, daß die Glühwendel der Strahlenquelle von der Kugelkalotte nicht auf sich selbst, sondern leicht versetzt abgebildet wird. Dadurch wird der Wirkungsgrad der Bestrahlungsvorrichtung auf hohem Niveau gehalten.

Im folgenden wird die Wirkungs- und Funktionsweise der erfindungsgemäßen Bestrahlungsvorrichtung näher erläutert. Der mit Wasser gefüllte Hohlraum 14 des Filters 3 bewirkt bei angeschalteter Strahlungsquelle, daß bestimmte Bereiche des Strahlenspektrums, die sog. Wasserbanden, herausgefiltert werden. Ein solches Strahlenspektrum eignet sich in besonderes Weise zur Behandlung des menschlichen Körpers, da eine Überwärmung bestimmter Körperregionen erreicht werden kann, ohne daß dies vom Patienten als schmerzhaft empfunden wird. Um eine größere Fläche gleichmäßig bestrahlen zu können, ist es erforderlich, eine entsprechend leistungsstarke Strahlungsquelle, z.B. eine Halogenlampe, zu verwenden. Eine solche leistungsstarke Strahlungsquelle 2 erwärmt naturgemäß den sich im Strahlungsgang befindenden Wasserfilter 3. Durch diese Erwärmung dehnt sich zum einen das Medium in dem Hohlraum 14 aus; zum anderen muß dafür gesorgt werden, daß die Erwärmung des Wasserfilters 3 in solchen Grenzen gehalten wird, daß keine Schädigung des Wasserfilters herbeigeführt wird. Der Druckausgleich des Wasserfilters 3 wird auf einfache Weise dadurch bewerkstelligt, daß man den Hohlraum 14 mit einem Schlauch 18 aus einem aufweitbaren Material verbindet. Eine andere Alternative ist in Fig. 3 dargestellt. Dort ist eine Blase 17 in dem Hohlraum 14 vorgesehen, die mit Luft gefüllt ist. Die Blase 17 kann bei Ansteigen des Drucks im Hohlraum 14 komprimiert werden, so daß auch hierüber ein Druckausgleich stattfinden kann, ohne daß die Scheiben 4 oder 5 unzulässig hohen Spannungen ausgesetzt werden. Der Abtransport der in dem Medium befindlichen Wärme wird bei dem beschriebenen Ausführungsbeispiel auf besonders geschickte Weise dadurch gefördert, daß die Scheibe 5 topfförmig ausgebildet ist, so daß der Rahmen 6 eine größere Kontaktfläche zu dem in dem Hohlraum 14 befindlichen Medium aufweist. Obgleich zwar die Kontaktfläche, wie auch bei dem hier gezeigten Beispiel geschehen, durch das Vorsehen von Kühlrippen 15 vergrößert werden kann, bewirkt die topfförmige Ausgestaltung der Scheibe 5, daß der für den Wärmetransport zur Verfügung stehende Materialquerschnitt ebenfalls relativ groß gehalten werden kann, was den Wärmetransport nach außen erheblich begünstigt. Die Wärme wird dann letztlich über die äußeren Kühlrippen 9 an den durch den Kühlventilator 10 verursachten Luftstrom abgegeben.

Bei senkrechtem Betrieb der Vorrichtung ist es eventuell günstig, die beiden Scheiben leicht gewölbt auszubilden, um durch einen Höhenunterschied eine Konvektion des Mediums im Filter zu erleichtern.

## Patentansprüche

1. Bestrahlungsvorrichtung mit einer Strahlenquelle (2) und einem im Strahlengang angeordneten Filter (3), der zwei transparente, im wesentlichen planparallel zueinander angeordnete Scheiben (4, 5) aufweist, die mit ihren umlaufenden Rändern (7, 8) in einem Rahmen (6) aus gut wärmeleitendem Material gehalten sind, wobei die Scheiben (4, 5) und der Rahmen (6) einen geschlossenen Hohlraum (14) begrenzen, in dem ein das Strahlenspektrum selektiv beeinflussendes Medium vorgsehen ist, **dadurch gekennzeichnet,** daß zumindest eine der Scheiben (5) topfförmig mit einem abgestuften Rand (8) derart ausgebildet ist, daß der lichte Abstand (b) der im Rahmen (6) gehaltenen Ränder (7, 8) der Scheiben (4, 5) größer ist als der lichte Abstand (c) der mittleren Bereiche (11) der Scheiben (4, 5).

2. Bestrahlungsvorrichtung nach Anspruch 1, **dadurch gekennzeichnet,** daß die topfförmige Scheibe (5) auf der der Strahlenquelle (2) abgewandten Seite des Filters vorgesehen ist.

3. Bestrahlungsvorrichtung nach Anspruch 1 oder 2, **dadurch gekennzeichnet,** daß die topfförmige Scheibe (5) aus Kunststoff, vorzugsweise Polycarbonat, besteht.

4. Bestrahlungsvorrichtung nach einem der Ansprüche 1 bis 3, **dadurch gekennzeichnet,** daß die der Strahlenquelle (2) zugewandte Scheibe (4) aus einem hitzebeständigen Material, vorzugsweise Mineralglas, besteht.

5. Bestrahlungsvorrichtung nach einem der Ansprüche 1 bis 4, **dadurch gekennzeichnet,** daß die Stufe (12) der topfförmigen Scheibe (5) im wesentlichen kegelschnittförmig und zum Rand (8) hin beabstandet ausgebildet ist.

6. Bestrahlungsvorrichtung nach einem der Ansprüche 1 bis 5, **dadurch gekennzeichnet,** daß der Abstand (a) der Stufe (12) zum Rand (8) etwa zwischen 1/3 bis 1/10 des Radius (r) der Scheibe (5) beträgt.

7. Bestrahlungsvorrichtung nach einem der Ansprüche 1 bis 6, **dadurch gekennzeichnet,** daß das Verhältnis des lichten Abstandes (b) der Ränder (7, 8) zum lichten Abstand (c) der mittleren Bereiche (11) der Scheiben (4, 5) etwa zwischen 7:1 bis 3:1 beträgt.

8. Bestrahlungsvorrichtung nach einem der Ansprüche 1 bis 7, **dadurch gekennzeichnet,** daß der Rahmen (6) an seiner dem Hohlraum (14) zugewandten Seite mit in das Medium reichenden Kühlrippen (15) versehen ist.

9. Bestrahlungsvorrichtung nach einem der Ansprüche 1 bis 8, **dadurch gekennzeichnet,** daß die Strahlenquelle etwa im Brennpunkt eines paraboloidförmigen Reflektors (19) angeordnet ist, dessen Reflektorwände bis nahe an den Filter (3) heranreichen.

10. Bestrahlungsvorrichtung nach Anspruch 9, **dadurch gekennzeichnet,** daß der Reflektor (19) im Bereich der Strahlungsquelle (2) zylinderförmig und hinter der Strahlenquelle (2) kugelkalottenförmig ausgebildet ist.

11. Filter für eine Bestrahlungsvorrichtung nach einem der Ansprüche 1 bis 10, mit zwei transparenten, im wesentlichen planparallel angeordneten Scheiben (4, 5), die mit ihren umlaufenden Rändern (7, 8) in einem Rahmen (6) aus gut wärmeleitendem Material gehalten sind, wobei die Scheiben (4, 5) und der Rahmen (6) einen geschlossenen Hohlraum (14) begrenzen, in dem ein das Strahlenspektrum selektiv beeinflussendes Medium vorgesehen ist, **dadurch gekennzeichnet,** daß zumindest eine der Scheiben (5) topfförmig mit einem abgestuften Rand (8) derart ausgebildet ist, daß der lichte Abstand (b) der im Rahmen gehaltenen Ränder (7, 8) der Scheiben (4, 5) größer ist als der lichte Abstand (c) der mittleren Bereiche (11) der Scheiben (4, 5).

12. Filter nach Anspruch 11, der nach dem kennzeichnenden Teil eines der Ansprüche 2 bis 8 weitergebildet ist.

13. Filter nach Anspruch 11 oder 12, **dadurch gekennzeichnet,** daß der durch die Scheiben (4, 5) und den Rahmen (6) begrenzte Hohlraum (14) mit einer komprimierbaren oder expandierbaren Druckausgleichseinrichtung verbunden ist.

14. Filter nach Anspruch 13, **dadurch gekennzeichnet,** daß die Druckausgleichseinrichtung eine im Hohlraum (14) angeordnete, von einer Haut umgebene oder freie Blase (17) ist, die mit einem kompressiblen Medium gefüllt ist.

15. Filter nach Anspruch 13, **dadurch gekennzeichnet,** daß die Druckausgleichseinrichtung ein über eine Bohrung im Rahmen (6) mit dem Hohlraum (14) verbundener, geschlossener Schlauch (18) aus einem flexiblen Material ist.

16. Filter nach Anspruch 13, **dadurch gekennzeichnet,** daß der Rahmen (6) aus einem balgenartig geformten Material gebildet ist.

17. Filter nach einem der Ansprüche 11 bis 16, **dadurch gekennzeichnet,** daß die topfförmige Scheibe (5) als spektraler Kantenfilter eingefärbt ist.

18. Filter nach einem der Ansprüche 11 bis 17, **dadurch gekennzeichnet,** daß der mittlere Bereich (11) der topfförmigen Scheibe (5) als Fresnel-Scheibe ausgebildet ist.

19. Filter nach einem der Ansprüche 11 bis 18, **dadurch gekennzeichnet,** daß die der Strahlenquelle zugewandte Scheibe (4) des Filters mit einer das sichtbare Spektrum reflektierenden Bedampfungsschicht oder Folie versehen ist, die den Filter belastende thermische Strahlung mindert.

## Claims

1. Irradiating device having a radiation source (2) and a filter (3) which is disposed in the beam path and which exhibits two transparent plates (4, 5) which are disposed to be substantially plane-parallel to one another and which are held by their circulating margins (7, 8) in a frame (6) of material having good thermal conductivity, in which the plates (4, 5) and the frame (6) define a closed cavity (14), in which a medium selectively influencing the radiation spectrum is provided, characterized in that at least one of the plates (5) is designed to be pot-shaped with a stepped margin (8), in such a manner that the clear spacing (b) of those margins (7, 8) of the plates (4, 5) which are held in the frame (6) is greater than the clear spacing (c) of the central regions (11) of the plates (4, 5).

2. Irradiating device according to Claim 1, characterized in that the pot-shaped plate (5) is provided on that side of the filter which faces away from the radiation source (2).

3. Irradiating device according to Claim 1 or 2, characterized in that the pot-shaped plate (5) comprises plastic material, preferably polycarbonate.

4. Irradiating device according to one of Claims 1 to 3, characterized in that the plate (4) facing the radiation source (2) comprises a heat-resistant material, preferably mineral glass.

5. Irradiating device according to one of Claims 1 to 4, characterized in that the step (12) of the pot-shaped plate (5) is designed to be substantially conic-section-shaped and spaced off towards the margin (8).

6. Irradiating device according to one of Claims 1 to 5, characterized in that the spacing (a) of the step (12) from the margin (8) is approximately between 1/3 and 1/10 of the radius (r) of the plate (5).

7. Irradiating device according to one of Claims 1 to 6, characterized in that the ratio of the clear spacing (b) of the margins (7, 8) to the clear spacing (c) of the central regions (11) of the plates (4, 5) is approximately between 7:1 and 3:1.

8. Irradiating device according to one of Claims 1 to 7, characterized in that the frame (6) is provided, at its side facing towards the cavity (14), with cooling ribs (15) extending into the medium.

9. Irradiating device according to one of Claims 1 to 8, characterized in that the radiation source is disposed approximately at the focus of a paraboloid reflector (19), the reflector walls of which extend as far as close to the filter (3).

10. Irradiating device according to Claim 9, characterized in that the reflector (19) is designed to be cylindrical in the region of the radiation source (2) and spherical-segment-shaped behind the radiation source (2).

11. Filter for an irradiating device according to one of Claims 1 to 10, having two transparent plates (4, 5) which are disposed to be substantially plane-parallel and which are held by their circulating margins (7, 8) in a frame (6) of material of good thermal conductivity, in which the plates (4, 5) and the frame (6) define a closed cavity (14), in which a medium selectively influencing the radiation spectrum is provided, characterized in that at least one of the plates (5) is designed to be pot-shaped with a stepped margin (8), in such a manner that the clear spacing (b) of those margins (7, 8) of the plates (4, 5) which are held in the frame is greater than the clear spacing (c) of the central regions (11) of the plates (4, 5).

12. Filter according to Claim 11, which is further formed according to the characterizing clause of one of Claims 2 to 8.

13. Filter according to Claim 11 or 12, characterized in that the cavity (14) defined by the plates (4, 5) and the frame (6) is connected to a compressible or expansible pressure compensating device.

14. Filter according to Claim 13, characterized in that the pressure compensating device is a bubble (17) which is disposed in the cavity (14) and which is surrounded by a skin or free, and which is filled with a compressible medium.

15. Filter according to Claim 13, characterized in that the pressure compensating device is a closed hose (18) of a flexible material, which hose is connected to the cavity (14) via a bore in the frame (6).

16. Filter according to Claim 13, characterized in that the frame (6) is formed from a material shaped in the manner of a bellows.

17. Filter according to one of Claims 11 to 16, characterized in that the pot-shaped plate (5) is stained as a spectral cut-off filter.

18. Filter according to one of Claims 11 to 17, characterized in that the central region (11) of the pot-shaped plate (5) is designed as a Fresnel lens.

19. Filter according to one of Claims 11 to 18, characterized in that that plate (4) of the filter which faces towards the radiation source is provided with a vapour deposition layer or foil which reflects the visible spectrum and which reduces thermal radiation stressing the filter.

## Revendications

1. Dispositif d'irradiation avec une source de rayonnement (2) et un filtre (3) disposé sur le trajet des rayons, oui comporte deux disques (4, 5) transparents disposés sensiblement de façon plane et parallèles l'un par rapport à l'autre, et qui sont maintenus par leurs bords circonférentiels (7, 8) dans un cadre (6) en matière de bonne conduction thermique les disques (4, 5) et le cadre (6) délimitant un espace creux fermé (14) dans lequel est prévu un fluide influençant sélectivement le spectre de rayonnement, caractérisé en ce qu'au moins l'un des disques (5) est conçu sous la forme d'un pot avec un bord à gradin (8), de telle façon que l'écartement libre (b) entre les bords (7, 8) des disques (4, 5) maintenus dans le cadre (6) est supérieur à l'écartement libre (c) entre les zones centrales (11) des disques (4, 5).

2. Dispositif d'irradiation selon la revendication 1, caractérisé en ce que le disque en forme de pot (5) est prévu du coté du filtre opposé à la source de rayonnement (2).

3. Dispositif d'irradiation selon l'une des revendications 1 ou 2, caractérisé en ce que le disque en forme de pot (5) est en matière plastique, de préférence en polycarbonate.

4. Dispositif d'irradiation selon l'une quelconque des revendications 1 à 3, caractérisé en ce que le disque (4) faisant face à la source de rayonnement (2) est constitué d'une matière résistant à la chaleur, de préférence du verre minéral.

5. Dispositif d'irradiation selon l'une quelconque des revendications 1 à 4, caractérisé en ce que le gradin (12) du disque en forme de pot (5) est conçu avec une section sensiblement conique et présente un écartement bar rapport au bord (8).

6. Dispositif d'irradiation selon l'une quelconque des revendications 1 à 5, caractérisé en ce que l'écartement (a) entre le gradin (12) et le bord (8) est compris approximativement entre un 1/3 et 1/10ème du rayon (r) du disque (5).

7. Dispositif d'irradiation selon l'une quelconque des revendications 1 à 6, caractérisé en ce que le rapport de l'écartement libre (b) entre les bords (7, 8) et de l'écartement libre (c) entre les zones centrales (11) des disques (4, 5) est de l'ordre de 7:1 à 3:1.

8. Dispositif d'irradiation selon l'une quelconque des revendications 1 à 7, caractérisé en ce que le côté du cadre (6) faisant face à l'espace creux (14) est équipé d'ailettes de refroidissement (15) qui pénètrent dans le fluide.

9. Dispositif d'irradiation selon l'une quelconque des revendications 1 à 8, caractérisé en ce que la source de rayonnement est approximativement disposée au niveau du point focal d'un réflecteur (19) ayant la forme d'un paraboloïde, dont les parois de réflexion arrivent à proximité du filtre (3).

10. Dispositif d'irradiation selon la revendication 9, caractérisé en ce que, dans la zone de la source de rayonnement (2), le réflecteur (19) est conçu en forme de cylindre, et derrière la source de rayonnement (2), il est conçu en forme de calotte sphérique.

11. Filtre pour un dispositif d'irradiation selon l'une quelconque des revendications 1 à 10, comportant deux disques (4, 5) transparents disposés sensiblement de façon plane et parallèle, qui sont maintenus par leurs bords circonférentiels (7, 8) dans un cadre (6) en matière de bonne conduction thermique, les disques (4, 5) et le cadre (6) délimitant un espace creux fermé (14) dans lequel est prévu un fluide influençant sélectivement le spectre de rayonnement, caractérisé en ce qu'au moins l'un des disques (5) est conçu sous la forme d'un pot avec un bord à gradin (8), de telle façon que l'écartement libre (b) entre les bords (7, 8) des disques (4, 5) maintenus dans le cadre est supérieur à l'écartement libre (c) entre les zones centrales (11) des disques (4, 5).

12. Filtre selon la revendication 11, qui est perfectionné selon la partie caractérisante de l'une quelconque des revendications 2 à 8.

13. Filtre selon l'une des revendications 11 ou 12, caractérisé en ce que l'espace creux (14) délimité par les disques (4, 5) et le cadre (6) est relié à un dispositif de compensation de pression compressible ou expansible.

14. Filtre selon la revendication 13, caractérisé en ce que le dispositif de compensation de pression est une bulle (17) libre ou entourée d'une pellicule disposée dans l'espace creux (14), laquelle est remplie d'un fluide compressible.

15. Filtre selon la revendications 13, caractérisé en ce que le dispositif de compensation de pression est un tuyau fermé (18) en matière flexible qui est relié à l'espace creux (14) par l'intermédiaire d'un perçage pratiqué dans le cadre (6).

16. Filtre selon la revendication 13, caractérisé en ce que le cadre (6) est constitué d'un matériel du type soufflet.

17. Filtre selon l'une quelconque des revendications 11 à 16, caractérisé en ce que le disque en forme de pot (5) est coloré en tant que filtre spectral à arêtes.

18. Filtre selon l'une quelconque des revendications 11 à 17, caractérisé en ce que la zone centrale (11) du disque en forme de pot (5) est conçue en tant que disque de Fresnel.

19. Filtre selon l'une quelconque des revendications 11 à 18, caractérisé en ce que le disque (4) du filtre faisant face à la source de rayonnement est revêtu d'une couche de métallisation ou d'un film reflétant le spectre lumineux, qui réduit le rayonnement thermique auquel est soumis le filtre.
